# EUROPEAN PATENT APPLICATION

(11) **EP 1 752 047 A2**
(43) Date of publication of application: **14.02.2007**
(21) Application number: 06290965.0
(22) Date of filing: 13.06.2006
(51) Int. Cl.: A23G 3/56, A23G 3/50, A23G 3/36, A61Q 1/04

(54) **Confectionary lip gloss and method of use**

(30) Priority: 05.08.2005 US 705916 P; 13.06.2006 US 451708 P
(71) Applicant: Home Focus Development Limited, Road Town, Tortola (VG)
(72) Inventor: Andersen, Jacob Ranis Stocholm, Hong Kong (CN)
(74) Representative: Breese Derambure Majerowicz

(57) **Abstract**

A confectionary lip gloss product is described. The product includes a vessel with a hollow cavity and an edible fluid confectionary housed within the vessel. An applicator for applying the fluid confectionary to a user's lips can also be provided. A method of consuming a confectionary is also described. The method involves the steps of applying an edible fluid confectionary to lips of a user in a substantially even and uniform manner. Once applied, the user can lick the edible fluid confectionary from the lips and ingest it. A method of marketing a confectionary product is also described. The marketing method involves packaging an edible fluid confectionary in a vessel, selling the edible fluid confectionary in combination with an applicator; and instructing a user to apply the fluid confectionary to a user's lips using the applicator.

## Description

### Field of the Invention

The invention relates to the field of confectionary products, and particularly to a confectionary product that simulates lip gloss and to a method of decorating one's lips with a confectionary.

### Background

Candy and other confectionaries have been known for centuries. Today candy is available in a variety of shapes, sizes and colors. Some candies are decorative, while others are plain. However, beyond taste or any decorative features, candy generally lacks entertaining or amusement qualities.

Conventionally, candy is sold in a disposable wrapper or other protective packaging that is removed before the user places the candy in his or her mouth. In some instances, the candy wrapper can include promotional or entertaining items. Still, the candy itself is usually meant to be simply removed from the wrapper and consumed.

What is needed is a way to add amusement qualities and value to candy, and to provide an additional incentive for the consumer to buy the candy.

### Summary of the Invention

This invention relates to a confectionary lip gloss product. The product includes an edible fluid confectionary housed within a vessel. Preferred embodiments of the edible fluid confectionary include syrup and a humectant. The vessel can include a removable cap and an applicator for applying the edible fluid confectionary to the user's lips.

The invention also relates to a method of consuming a confectionary. The method involves providing an edible fluid confectionary housed within a vessel. Preferred embodiments include an applicator in combination with the edible fluid confectionary. The user can apply the edible fluid confectionary to his or her lips with or without using the applicator. If desired, the user can then lick the edible fluid confectionary from the lips and ingest it.

The invention further relates to a method of marketing a confectionary product. The marketing method involves packaging an edible fluid confectionary in a vessel and selling the edible fluid confectionary in combination with an applicator. The method further includes the step of instructing a user to apply the edible fluid confectionary to the user's lips using the applicator.

### Brief Description of the Drawings

For the purpose of illustrating the invention, there are shown in the drawings forms which are presently preferred; it being understood, that this invention is not limited to the precise arrangements and instrumentalities shown.

Fig. 1 is a view of a vessel, removable cap and applicator according to an embodiment of the invention.

Fig. 2 is a longitudinal cross sectional view of a confectionary lip gloss product according to an embodiment of the invention.

### Detailed Description

It will be appreciated that the following description is intended to refer to specific embodiments of the invention selected for illustration in the drawings and is not intended to define or limit the invention, other than in the appended claims.

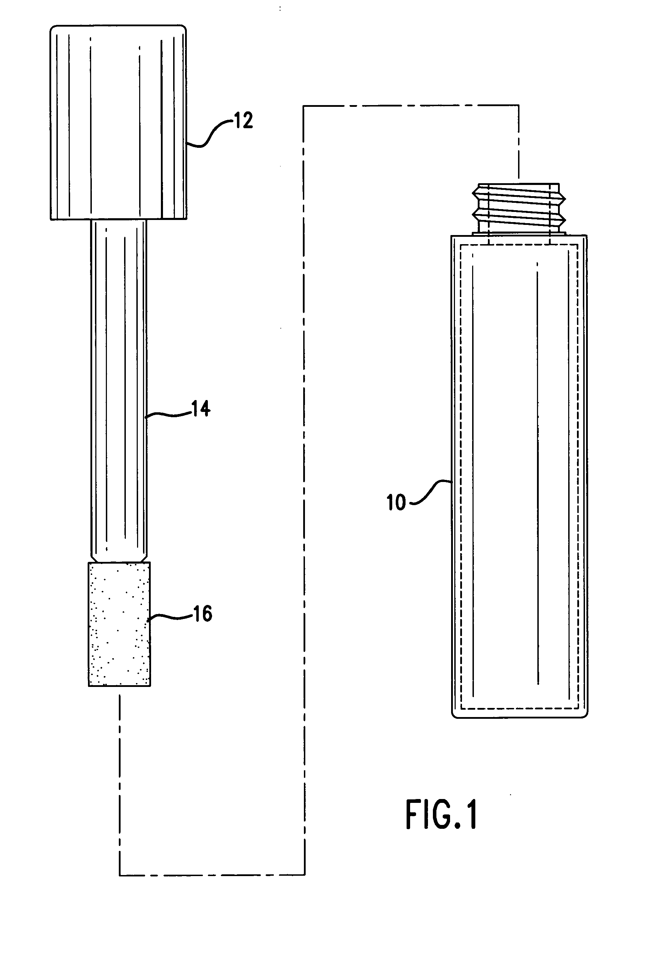

In the figures, in which like reference numerals indicate like elements, there are shown preferred embodiments of a confectionary lip gloss product. Figure 1 shows a preferred embodiment of a container that can be used in connection with the invention. The container includes a vessel 10, a removable cap 12 and an applicator 14. Flocking material 16 is disposed on the tip of the applicator tip 14. The vessel 10, cap 12 and applicator 14 are preferably molded from a plastic material, such as polypropylene. However, any other suitable rigid or flexible material could also be used, such as metal, wood, *etc*.

The flocking material 16 can be any synthetic or natural material that can absorb or otherwise hold fluid and then release the fluid when contacted with or placed adjacent to a target surface. Materials, other than flocking material, can alternatively be used at the tip of the applicator 14. For example, a brush or a tube designed to fill with fluid and then release the fluid could also be used. Collectively, all of these materials (flocking material, brushes, tubes and other suitable materials) can be referred to as fluid delivering materials. The applicator can alternatively be in the form of a "roll-on" delivery device or a transfer member with a fluid-delivery surface. Such a transfer member can be a plain post, such as an elongated cylinder, prism or flattened element. The end of the plain post can serve as the fluid-delivery surface, and can have the same transverse cross section as the rest of the post, a wider cross section or a narrower cross section. The fluid delivery surface could also be concave, grooved or otherwise specially adapted for fluid transfer.

Preferably, the applicator 14 is formed with the removable cap 12 so that the applicator 14 is disposed within the vessel 10 when the cap 12 is engaged therewith. To accomplish this, the applicator 14 can be molded integrally with the cap 12 or the applicator can be a separate piece that is attached to the inside of cap 12 with a friction set, glue or mechanical means. The inside surface of the cap 12 can be provided with female threads that correspond with male threads on the vessel 10 for engagement therewith. However, other means of removably engaging cap 12 with vessel 10 can instead be employed. Such means would be apparent to those skilled in the art, and can include snap fits, bayonet fits, friction fits and the like.

The shape of the vessel 10 is preferably cylindrical as shown, to simulate a conventional lip gloss container. However, the vessel can be made in any suitable shape, for example in the shape of a perfume bottle or cosmetic product, stylized human being or animal, or in a fanciful shape. The vessel can also be made in the likeness (including a caricature) of an actual sports figure. The vessel can also comprise one or more colors, designs or indicia indicating a player or team for use in a game, and can itself be used as a game piece.

The vessel 10 is designed to house an edible fluid confectionary. The edible fluid confectionary can be a flowable material. Such materials can include emulsions, suspensions, solutions, *etc.* Other suitable materials can include creams, pastes, jellies, powders (especially conglomerated powders), *etc.*

Preferably, the edible fluid confectionary is syrup based. As used herein, the term "syrup" means a viscous and edible liquid that contains a substantial amount of dissolved sugars or artificial sweeteners. When the syrup contains sugar, the sugars should not deposit crystals to any significant extent. Suitable syrups include corn syrup (including high fructose corn syrup), fruit syrup, honey, inverted sugar syrup, maple syrup, sugar beet syrup and squash-type syrups. Other suitable syrups are those that include artificial sweeteners that will participate in polar or other interactions in solution to impart the required viscosity, or other artificial sweeteners if viscosity enhancers are included to increase viscosity sufficiently. Of course, it should be understood that viscosity enhancers can be used in any syrup if desired.

The edible fluid confectionary can include more than about 40% by weight of syrup. Preferably, the content of syrup is between 45% and 60% by weight and more preferably about 50% to about 55% by weight. When the syrup is corn syrup, it is most preferably present at about 53% by weight. However, the syrup content can be increased when less viscous syrup is used, and can be decreased when syrup of greater viscousity is used or when a high sugar content (described below) is provided.

It should be understood that the syrup can be, but need not be, added in syrup form to make the edible fluid confectionary. Instead, the syrup can alternatively be produced as the edible fluid confectionary is made, for example, by boiling or evaporating juices or other aqueous solutions until the proper viscosity is obtained, by enzymatic action on starches (such as used in producing high fructose corn syrup) or by dissolving sugar in water to the saturation point and then evaporating the excess water under conditions that will produce syrup. Syrup could also be produced with artificial sweetener, with or without a viscosity enhancer, as needed.

The edible fluid confectionary can include a humectant, with glycerin being a preferred humectant. Glycerin is also known as glycerol and glycerine, as well as by other less common names. The humectant helps maintain appropriate moisture levels when the product is stored, handled and used. Further, the humectant, especially glycerin, can act as a stabilizer to enhance shelf life of the product, and/or act as a viscosity modifier. Other humectants suitable for use in the invention can include, but are not limited to, mannitol, sorbitol, fructose, and propylene glycol. The humectant can be present in an amount of between about 5% and about 15% by weight, more preferably between about 5% and about 10% by weight. When glycerin is used as the humectant, it is most preferably present at about 6% by weight.

The edible fluid confectionary can include sugar or one or more artificial sweeteners, in addition to that in the syrup. Preferred sugars are those substances which are based on simple crystalline mono- and di-saccharide structures, i.e., those which are based on C₄, C₅ (pentose) and C₆ (hexose) sugar structures. Preferred monosacharides include glucose and fructose. Preferred disaccharides include sucrose, lactose, maltose, *etc*. Among these, sucrose is most preferred. Other hexose monosaccharides can include allose, altrose, mannose, gulose, idose, galactose, talose, psicose, verbose and tagatose. Pentose saccharides include ribose, arabinose, xylose, lyxose, ribulose and xylulose. Four carbon saccharides include erythrose, threose and erythrulose. Other disaccharides include isomaltose and cellobiose. Other sugars can include tri-, tetra- and oligosaccharides. The term "sugars" should also be understood to include sugar alcohols such as sorbitol, mannitol, maltitol, etc. Sugars can be present in the edible fluid confectionary in an amount of between about 5% and about 15% by weight (in addition to the sugar from the syrup component), more preferably between about 7% and 12% by weight. When sucrose is the sugar, it is most preferably provided at about 9% by weight.

The edible fluid confectionary can include artificial sweeteners instead of, or in addition to, sugar. Suitable artificial sweeteners can include aspartame, acesulfame, neotame, sucralose, saccharin, cyclamate and alitame. The amounts of artificial sweeteners to be used as sugar substitutes can be readily determined from the literature. For example, certain artificial sweeteners are 200 times sweeter than sugar, while others are more or less so. If artificial sweeteners that do not participate in hydrogen bonding are used, it may be desirable to add additional viscosity enhancers to ensure that the edible fluid confectionary can coat lips in the desired fashion, as is described below.

The edible fluid confectionary can include minor proportions of preservatives, such as citric acid (preferably between about 1.5% and about 3% by weight) or sodium benzoate (preferably between about 0.05% and about 0.12%), flavoring (artificial or otherwise, preferably between about 0.5% and about 1%), coloring (such as artificial color) and other additives that can enhance the color, texture, taste, viscosity, processability or shelf life of the confectionary. Flavoring can include any flavor that may appeal to a user, including fruit flavors, such as strawberry or cherry, bubblegum, cola, *etc*. The edible fluid confectionary can also include a substantial water component, preferably between about 15% and about 50% by weight, more preferably between about 20% and about 40%, most preferably between about 25% and about 30%. A preferred composition for the edible fluid confectionary is shown below in Table 1, where each component is shown by weight percent.

**TABLE 1**

| COMPONENT | WEIGHT PERCENT |
|---|---|
| Corn Syrup | 53.3% |
| Water | 28.5% |
| Sugar | 8.7% |
| Glycerin | 6.4% |
| Citric Acid | 2.3% |
| Cherry Artificial Flavor | 0.7% |
| Sodium Benzoate | 0.8% |
| Artificial Color E 129 | 0.02% |

Another preferred formulation for the edible fluid confectionary is shown below in Table 2.

**TABLE 2**

| COMPONENT | WEIGHT PERCENT |
|---|---|
| Corn Syrup | 53.3% |
| Water | 28.515% |
| Sugar | 8.7% |
| Glycerin | 6.4% |
| Citric Acid | 2.3% |
| Strawberry Artificial Flavor | 0.7% |
| Sodium Benzoate | 0.8% |
| Artificial Color E129 | 0.005% |

The edible fluid confectionary is formulated so that it can be eaten in substantial quantities. For this reason, it is important to a avoid using ingredients that will sicken a user if consumed in substantial quantities. Therefore, certain components of conventional lip gloss and lip balms should be avoided. However, the formulations of the invention provide the edible fluid confectionary with the ability to simulate the appearance of lip gloss when the edible fluid confectionary is applied to the user's lips. To properly simulate lip gloss, the edible fluid confectionary, when applied, can exhibit a smooth, glossy and/or shiny appearance. If desired, small, edible, reflective, candy-based or non-candy-based solids, such as flakes, can be added to the edible fluid confectionary to create a sparkling effect.

In addition to the precise visual effects desired, care should be taken to ensure that the edible fluid confectionary has a sufficient viscosity so that it can be used to coat lips in a substantially even and uniform manner. Based on the description herein, one skilled in the art would understand that various modifications to the above formulations can be made while still maintaining the desired edibility, coatability and appearance.

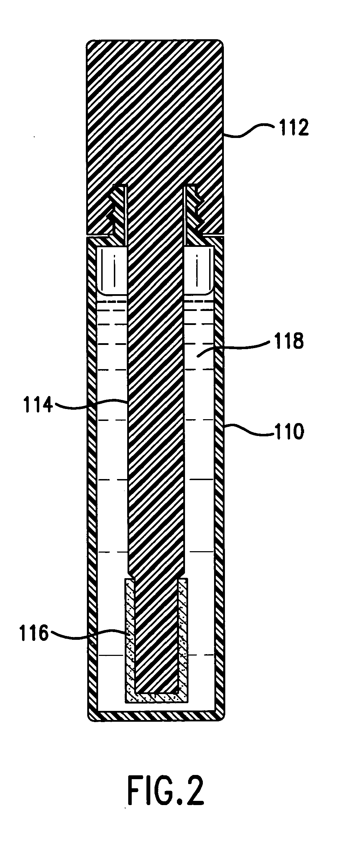

Fig. 2 shows a longitudinal cross section of an exemplary confectionary lip gloss product. The product contains a vessel 110, a removable cap 112 and an applicator 114. Disposed on the end of the applicator 114 is flocking material 116. An edible fluid confectionary 118 is housed within the vessel 110. The edible fluid confectionary can be as described above. As shown in Fig. 2, the cap 112 is threadedly engaged with the neck of the vessel 110. In this configuration, the applicator 114 is disposed within the vessel 110 in contact with the edible fluid confectionary 118.

In use, the user can disengage the cap 112 from the vessel 110 by unscrewing the cap from its threaded engagement. Thereafter, as the user removes the cap 112, the applicator 114 is withdrawn from the interior of the vessel 110. Upon complete removal of the applicator 114, it is evident to the user that the flocking material 116 is at least partially saturated with the edible fluid confectionary. Preferably, the edible fluid confectionary is sufficiently absorbed or otherwise held by the flocking material 116, such that the confectionary does not drip onto the user or the surrounding area once it is withdrawn from the vessel 110.

The user can apply the edible fluid confectionary to the user's lips by contacting the end of the applicator 114 and flocking material 116 with the user's lips and moving the end of the applicator 114 and flocking material 116 across the lips. Contacting the flocking material with the user's lips can sufficiently squeeze the flocking material 116 so as to release the edible fluid confectionary at the target location and coat the edible fluid confectionary onto the user's lips. As noted above, the same effect can be achieved by providing a brush or other fluid delivering material on the tip of the applicator 14.

To assist the user in properly operating the product, instructions can be provided that correlate the use of the applicator to decoration of the user's lips using the edible fluid confectionary. The instructions can instruct the user to coat the surface of the user's lips, preferably in an even and uniform manner. What is meant by coating or applying in an even and uniform manner is that the edible fluid confectionary is intentionally coated (as opposed to inadvertent deposition by incidental contact while consuming a confectionary) onto a substantial portion of the lips using deliberate brushing or other application techniques so as to provide a smooth lip-gloss type appearance. The instructions can include methods of applying the edible fluid confectionary using longitudinal strokes along the length of the lips, transverse strokes or combinations of these. The instructions can include methods of using the applicator to apply the edible fluid confectionary to both lips, as well as methods of applying to just one lip. If only one lip is decorated, some of the edible fluid confectionary can be transferred to the other lip by contacting the lips together.

If, after initial application, it is desired to coat additional amounts of edible fluid confectionary onto the user's lips, the user may reinsert the applicator into the vessel 110 to re-saturate the flocking material 116. The user may then withdraw the applicator 114 and contact the flocking material 116 with the user's lips in order to apply additional edible fluid confectionary. This process may be repeated as often as needed. When desired, the user can lick the edible fluid confectionary from the lips and ingest it. The user may also ingest the edible fluid confectionary directly.

In some embodiments, an applicator need not be provided with the vessel. In this case, the user can use an object that may be at hand, such as his or fingers, as the applicator. In such embodiments, the vessel can be in the form of a well, bowl or pot with a relatively large opening. The user can dip the object in the well and transfer a portion of the edible fluid confectionary to the lips. In the case where the user's finger is utilized, the finger tip can act as a fluid transfer surface.

In the case of two users who are romantically involved (or who wish to be), it is also possible that the edible fluid confectionary can be applied by either user to the lips of the first user. The edible fluid confectionary can then be transferred to the lips of the second user, such as by kissing. The second user can then lick the edible fluid confectionary from the lips and ingest it. Other methods of transferring and ingesting the edible fluid confectionary will be apparent to those skilled in the art.

The invention also relates to a method of marketing a confectionary product. The marketing method involves packaging an edible fluid confectionary in a vessel, such as one of the vessels shown in Figs. 1 or 2 or a similar vessel. The edible fluid confectionary can then be sold in combination with an applicator, such as those described above. Further, the user can be instructed to apply the edible fluid confectionary to the user's lips using the applicator.

As noted above, the instructions can instruct the user to coat the entirety of the surface of the user's lips, preferably in an even and uniform manner. The instructions can include methods of applying the edible fluid confectionary using longitudinal strokes along the length of the lips, transverse strokes or combinations of these. The methods of application can also be advertised by methods known to those skilled in the art to inform potential users of the confectionary lip gloss product and to entice them to purchase it.

The confectionary lip gloss product can also be integrated into a larger marketing scheme or with other products. If the confectionary lip gloss product includes a vessel shaped as a game piece, for example, the product can include other items, such as a foldable housing to contain the playing pieces and/or game board when not in use, scorecards or other devices to record game statistics and results. The confectionary lip gloss product can also include candy or gum, electronic devices (such as for producing light and sound effects or play-by-play announcements during game play), suggested rules of play, a timing device, stickers or the like for decorating the game pieces and/or game board, and promotional items such as contests or lotteries and team or league paraphernalia.

A variety of modifications to the embodiments described will be apparent to those skilled in the art from the disclosure provided herein. Thus, the invention may be embodied in other specific forms without departing from the spirit or essential attributes thereof and, accordingly, reference should be made to the appended claims, rather than to the foregoing specification, as indicating the scope of the invention.

## Claims

1. A confectionary lip gloss product comprising:
a vessel with a hollow cavity;
an edible fluid confectionary housed within the vessel, the edible fluid confectionary comprising syrup and a humectant;
a removable cap engaged with the vessel; and
an applicator for applying the edible fluid confectionary to a user's lips.

2. The confectionary lip gloss product of claim 1, further comprising instructions that correlate the use of the applicator to decoration of the user's lips using the edible fluid confectionary.

3. The confectionary lip gloss product of claim 1 wherein, the instructions instruct the user to coat a surface of the user's lips in an even and uniform manner.

4. The confectionary lip gloss product of claim 1 wherein, the edible fluid confectionary further comprises sugar or an artificial sweetener.

5. The confectionary lip gloss product of claim 4 wherein, the edible fluid confectionary comprises a disaccharide.

6. The confectionary lip gloss product of claim 1 wherein, the edible fluid confectionary further comprises cherry flavoring.

7. The confectionary lip gloss product of claim 1 wherein, the edible fluid confectionary further comprises strawberry flavoring.

8. The confectionary lip gloss product of claim 1 wherein, the humectant is glycerin.

9. The confectionary lip gloss product of claim 1 wherein, the edible fluid confectionary comprises more than about 40% syrup by weight.

10. The confectionary lip gloss product of claim 9 wherein, the edible fluid confectionary comprises between about 50% and about 55% syrup by weight.

11. The confectionary lip gloss product of claim 9 wherein, the syrup is corn syrup.

12. The confectionary lip gloss product of claim 1 wherein, the edible fluid confectionary further comprises citric acid.

13. The confectionary lip gloss product of claim 12 wherein, the edible fluid confectionary further comprises a preservative.

14. The confectionary lip gloss product of claim 13 wherein, the edible fluid confectionary further comprises sugar.

15. The confectionary lip gloss product of claim 14 wherein, the humectant comprises glycerin, the syrup comprises corn syrup, the preservative comprises sodium benzoate, and the edible fluid confectionary comprises by weight between about 50% and about 55% corn syrup, between about 5% and about 10% sugar in addition to sugar in the corn syrup, between about 5% and about 15% glycerin, between about 1.5% and about 3% citric acid, between about 0.5% and about 1% flavoring and between about 0.05% and about 0.12% sodium benzoate.

16. A method of consuming a confectionary comprising:
providing an edible fluid confectionary housed within a vessel;
applying the edible fluid confectionary to lips of a user in a substantially even and uniform manner;
licking the fluid confectionary from the lips; and
ingesting the fluid confectionary.

17. The method of claim 16 wherein, the providing step comprises the step of including an applicator in combination with the edible fluid confectionary, and the applying step comprises using the applicator to apply the edible fluid confectionary in a substantially even and uniform manner.

18. The method of claim 17 further comprising the step of following instructions that correlate the use of the applicator to decoration of the user's lips using the edible fluid confectionary.

19. The method of claim 16 wherein, the providing step comprises the step of including instructions that instruct the user to apply the edible fluid confectionary to the user's lips.

20. The method of claim 16 wherein, the providing step comprises the step of combining syrup and a humectant.

21. The method of claim 18 wherein, the humectant is glycerin.

22. A method of marketing a confectionary product, the method comprising the steps of:
packaging an edible fluid confectionary in a vessel;
selling the edible fluid confectionary in combination with an applicator; and
instructing a user to apply the edible fluid confectionary to the user's lips using the applicator.

23. The method of claim 22 wherein, the instructing step comprises instructing the user to decorate the user's lips by applying the edible fluid confectionary in a substantially even and uniform manner.

24. The method of claim 22 wherein, the edible fluid confectionary is advertised or sold in combination with a foldable housing, a device to record game statistics or results, candy, gum, an electronic device, suggested rules of play, a timing device, stickers or promotional items.

25. A confectionary lip gloss product comprising:
a vessel with a hollow cavity;
an edible fluid confectionary housed within the vessel;
a removable cap engaged with the vessel; and
instructions that instruct a user to apply the edible fluid confectionary to the user's lips.

26. The confectionary lip gloss product of claim 25 further comprising an applicator for applying the edible fluid confectionary to the user's lips.

27. The confectionary lip gloss product of claim 25 wherein, the edible fluid confectionary comprises syrup and a humectant.
